# FASCICULE DE BREVET EUROPEEN

(11) **EP 0 694 615 B1**
(45) Date de publication et mention de la délivrance du brevet: **18.09.2002**
(21) Numéro de dépôt: 95401795.0
(22) Date de dépôt: 28.07.1995
(51) Int. Cl.: C12P 17/04

(54) **Méthode de préparation de gamma-nonalactones sous forme naturelle**
Verfahren zur Herstellung von Gamma-Nonalaktonen in natürlicher Form
Process for the preparation of gamma-nonalactones in native form

(30) Priorité: 28.07.1994 IT TO940628
(43) Date de publication de la demande: 31.01.1996
(73) Titulaire: San Giorgio Flavors S.p.A., 10147 Torino (IT)
(72) Inventeur: Allegrone, Gianna, I-10141 Torino (IT); Barbeni, Massimo, I-10141 Torino (IT); Carbella, Paolo, I-10128 Torino (IT); Villa, Mario, I-13058 Ponderano (BI) (IT); Fuganti, Claudio, I-20129 Milano (IT); Zucchi, Gioia, I-22030 Oliveto Lario (LC) (IT)
(74) Mandataire: Texier, Christian

(56) Documents cités:
- EP-A- 0 356 291
- EP-A- 0 412 880
- EP-A- 0 578 388
- TETRAHEDRON LETTERS, vol. 34, no. 40, 1993, OXFORD GB, pages 6467-6470, XP002048828 GIOVANNI FRONZA ET AL.: "On the mode of baker's yeast reduction of C-7 --- C-10 2-alken-4-olides"
- DATABASE WPI Section Ch, Week 8019 Derwent Publications Ltd., London, GB; Class B05, AN 80-33646C XP002048832 & JP 55 043 052 A (SUMITOMO CHEM CO LTD) , 26 Mars 1980
- CHEMICAL ABSTRACTS, vol. 121, no. 1, 4 Juillet 1994 Columbus, Ohio, US; abstract no. 4720, FUGANTI, CLAUDIO ET AL: "On the microbial biogeneration of (R).gamma.-jasmolactone" XP002048830 & BIOORG. MED. CHEM. LETT. (1993), 3(12), 2777-80 CODEN: BMCLE8;ISSN: 0960-894X,
- CHEMICAL ABSTRACTS, vol. 118, no. 3, 18 Janvier 1993 Columbus, Ohio, US; abstract no. 18985, ERCOLI, BARBARA ET AL: "Stereochemistry of the biogeneration of C-10 and C-12 gamma lactones in Yarrowia lipolytica and Pichia ohmeri" XP002048831 & BIOTECHNOL. LETT. (1992), 14(8), 665-8 CODEN: BILED3;ISSN: 0141-5492,
- CHEMICAL ABSTRACTS, vol. 122, no. 5, 30 Janvier 1995 Columbus, Ohio, US; abstract no. 50925, FUGANTI, CLAUDIO ET AL: "Biogeneration of gamma nonanolide from coriolic acid in Pichia stipitis and Pichia ohmeri" XP002048836 & BIOTECHNOL. LETT. (1994), 16(9), 935-8 CODEN: BILED3;ISSN: 0141-5492,

## Description

La présente invention concerne une méthode de préparation de gamma-lactones saturées ou insaturées ayant neuf atomes de carbone.

La récente introduction d'une distinction légale entre les arômes qui sont naturels et ceux ayant une structuré chimique identique mais qui sont synthétisés (Code américain des Réglementations fédérales 21: 101.22.a.3, Directive CEE 88/388 et D.L. 25 janvier 1992 N° 107) fait qu'il est devenu intéressant de produire de grandes quantités d'agents aromatisants non accessibles en les extrayant à partir de sources naturelles par biodégradation de produits naturels (D.W. Armstrong dans Flavor Chemistry, Trends and Developments, Ed. R. Teranishi, R.G. Buttery, F. Shahidi ; ACS Symposium Series 383, American Chemical Society, Washington D.C., 1989, p. 105-120). Ces produits sont préférés par le consommateur et ajoutent de la valeur aux aliments auxquels ils sont ajoutés ; il est par conséquent souhaitable qu'ils soient produits par ces méthodes.

Quelques-uns de ces produits aromatisants les plus intéressants sont les gamma- et delta-lactones en C₆ à C₁₂, qui peuvent être soit saturées soit mono- ou poly-insaturées, l'insaturation du cycle étant en diverses positions dans la chaîne. Il s'agit des constituants clés de beaucoup d'aromatisants de fruits et de produits laitiers. Elles sont générées dans les fruits en quantités extrêmement faibles, généralement lors du mûrissement. Il n'est pas économique d'extraire des lactones en vue d'une utilisation en tant qu'aromatisants à partir de sources naturelles en raison de la faible concentration à laquelle elles sont présentes, et parce que la concentration est fonction du degré de mûrissement et de beaucoup d'autres facteurs difficiles à contrôler. Ainsi, au cours des dernières années, pour satisfaire la demande en lactones naturelles, de nombreux procédés microbiologiques ont été mis au point dans lesquels les précurseurs naturels de lactones, comprenant les dérivés hydroxylés des acides gras naturels, sont dégradés en gamma- et delta-lactones selon la position de l'hydroxyle par rapport au carboxyle dans le précurseur mis en oeuvre.

Le gamma-décanolide est produit de cette manière à partir de l'acide ricinoléique (le brevet US 4.560.656 et le brevet européen EP-B-0.258.993). D'autres lactones ayant un nombre pair d'atomes de carbone sont obtenues par biodégradation microbienne des produits de photo-oxydation/réduction des acides oléique, linoléique et linolénique comme décrit dans EP 90402217.5.

Dans tous ces cas, les lactones obtenues contiennent un nombre pair d'atomes de carbone. Ceci est dû à des facteurs affectant la biosynthèse. Les précurseurs naturels hydroxylés obtenus à partir des acides insaturés en C₁₈ ou C₁₆ ont un nombre pair d'atomes de carbone et, si la dégradation est effectuée par béta-oxydation avec élimination de deux atomes de carbone sous forme de S-acétyle CoA, on obtient des lactones ayant un nombre pair d'atomes de carbone.

Le schéma de dégradation proposé est le suivant :

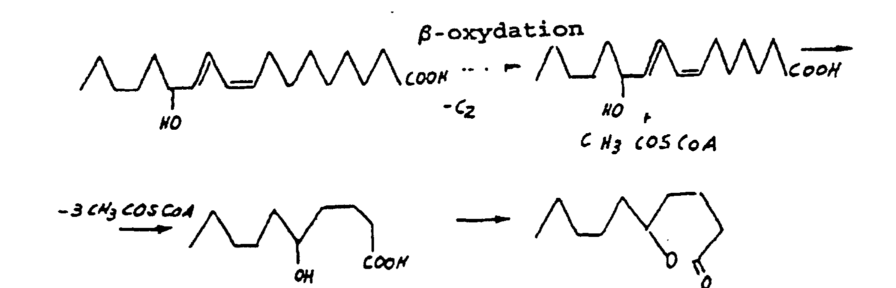

Toutefois, des gamma- et delta-lactones ayant un nombre impair d'atomes de carbone existent dans la nature. Parmi celles-ci le gamma-nonanolide (1) et le gamma-2-nonénolide (2) sont particulièrement intéressants ; leurs formules développées sont présentées ci-dessous :

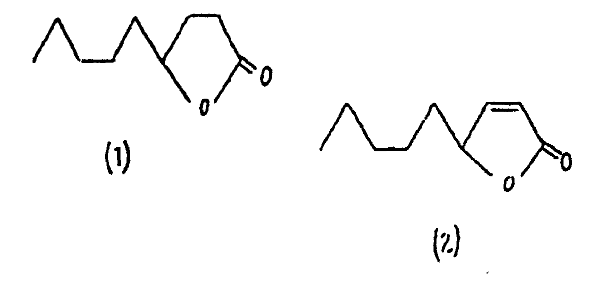

Ces deux produits sont des constituants mineurs de Dipteryx odorata (fève tonka) (M. Woerner et al. dans Unters. Forsch. 1991, 193, 21). La gamma-nonalactone est également un constituant connu des abricots, des fraises, des mûres, des pêches et de beaucoup d'autres fruits. Dans la forme racémique de synthèse, le gamma-nonanolide est largement utilisé dans l'industrie des aromatisants alimentaires, par exemple comme constituant des arômes de fruits. Toutefois, en dépit de l'intérêt de ces lactones à nombre impair d'atomes de carbone, il n'existe pas de procédés naturels capables de fournir des quantités importantes de produit sous une forme optiquement active.

La présente invention repose sur la découverte selon laquelle les lactones ayant neuf atomes de carbone saturés ou insaturés peuvent être obtenues à partir de précurseurs naturels par des procédés enzymatiques.

Fuganti, C. et al, Bioorg. Med, Chem. Lett., vol 3, no 12, 1993, p 2777 - 2780 montre qu'il est possible de préparer une γ-nonalactone avec une culture de Pichia ohmeri, mais pas avec Pichia stipitis. Or, contrairement à ce document, on a trouvé qu'il est possible de préparer un tel composé avec une culture de Pichia stipitis

Ainsi, l'objet de la présenté invention est une méthode de préparation de gamma-nonalactones saturées ou insaturées ou de leurs mélanges, caractérisée en ce qu'elle implique la culture de Pichia stipitis dans un substrat comprenant un hydroxy-acide insaturé en C₁₈, l'hydroxyle étant en C₁₃, ou un glycéride le contenant, pour obtenir un mélange comprenant le gamma-nonanolide ;

En référence à la variante au procédé ci-dessus, l'utilisation d'acide coriolique (R) est particulièrement préférée. En particulier, il a été trouvé que la dégradation microbiologique de l'acide coriolique (3), lorsqu' un micro-organisme tel que Pichia stipitis est utilisé, conduit à un mélange de gamma-nonanolide (1) et de delta-décanolide (4), vraisemblablement selon la chaîne réactionnelle ci-dessous :

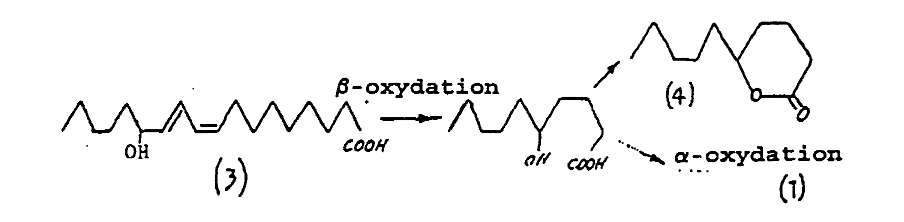

Il a été trouvé, de manière tout à fait inattendue, que le rendement en gamma-nonanolide (1) par rapport à celui en delta-décanolide (4) est beaucoup plus élevé lorsque la forme énantiomère (R) de l'acide coriolique (3) est utilisée. L'analyse par CGL chirale multidimensionnelle indique que le gamma-nonanolide (1) produit de cette manière comprend principalement l'énantiomère (R), tout comme le delta - décanolide (4) qui l'accompagne. L'acide coriolique (R) destiné à être utilisé comme substrat est susceptible d'être facilement obtenu à partir des graines oléagineuses de végétaux tels que ceux du genre Coriaria.

Lorsque l'acide coriolique (S) est utilisé comme substrat, le rendement en gamma-nonanolide (1) dans la forme (S) est bien inférieur.

D'autres substrats qui conviennent pour la préparation de gamma-nonanolide sont des mélanges d'hydroxy-acides contenant de l'acide coriolique obtenu par photo-oxydation/réduction ou lipoxygénation/réduction d'acides gras saturés ou insaturés en C₁₈ comprenant l'acide linoléique, d'acide linoléique seul ou en mélange avec d'autres acides gras insaturés tels que l'acide oléique. Toutefois, le rapport de gamma-nonanolide à d'autres lactones produites lorsque les micro-organismes ci-dessus sont cultivés dans ces substrats, dont la plus abondante est le delta-décanolide, est relativement faible. Ceci rend le procédé moins favorable dans la mesure où il est nécessaire de séparer le produit recherché des autres lactones.

Le procédé de photo-oxygénation est connu en soit et il est généralement réalisé dans un solvant en présence d'un photo-activateur naturel, comme décrit en détail dans la méthode, par exemple dans "The Lipid Handbook", Chapman & Holl, Londres, 1986, p. 453.

Le procédé de lipoxygénation, qui est connu en soi, est effectué avec de la lipoxygénase naturelle, de préférence avec le système enzymatique présent dans la farine de lin.

La réduction du produit de photo-oxydation ou de lipoxygénation pour produire les hydroxy-acides est de préférence effectuée en le traitant avec un agent de réduction naturel, tel que le sulfate ferreux ou la cystéine ou ses sels, dans un solvant approprié, par la méthode décrite par H.D. Belitz et W. Grosch dans Food Chemistry, Springer Verlag, Berlin, 1987, p. 172 ou H.W. Gardner, J. Agr. Food Chem. 1975, 23, 129.

Les hydroxy-acides peuvent être présents dans les mélanges ci-dessus à l'état d'acides libres, d'esters ou de glycérides.

En dehors du fait qu'ils présentent un intérêt particulier pour la production d'importantes quantités de gamma-nonanolide, les procédés décrits ci-dessus conduisent également à des mélanges de lactones naturelles, pouvant être utilisés comme constituants aromatisants. La composition de ces mélanges est fonction du micro-organisme, du substrat et du temps d'incubation. Ces mélanges peuvent alors être utilisés comme aromatisants ou peuvent être encore traités par des méthodes classiques pour séparer les composés souhaités.

### Exemples I-IV :

### Protocole général de biotransformation chez Pichia ohmeri et stipitis

Un échantillon de P.ohmeri (CSB 5367) et/ou de P.stipitis (CBS 5773) cultivé sur du MPGA (extrait de malt 20 g/l, peptone 5 g/l, glucose 20 g/l, agarose 15 g/l, pH 6,5-7) pendant trois jours à 28-30°C est utilisé.

Ce dernier est inoculé dans une fiole conique à fond plat de 300 ml contenant 50 ml de MPGB (extrait de malt 20 g/l, peptone 5 g/l, glucose 20 g/l). La fiole est placée sur un dispositif d'agitation (120 t/min) à 28-30°C pendant 24 heures. Le pre-inoculum résultant est inoculé dans un rapport de 10 % dans des fioles de 300 ml contenant 50 ml de MPGB et on laisse la croissance se poursuivre dans les mêmes conditions pendant 24 heures. A ce point, les substrats hydroxy-acides sont ajoutés dans une concentration de 2,5 g/l. On laisse la biotransformation se poursuivre dans les conditions de température et d'agitation décrites ci-dessus pendant respectivement 24 heures et 48 heures. A la fin de ces périodes, le mycélium est filtré, en éluant plusieurs fois sur le filtre avec du chlorure de méthylène. Ceci est alors utilisé pour extraire le liquide, dont le pH est augmenté à 4 par l'addition de solution d'acide citrique.

Les substrats utilisés étaient :
(a) l'acide coriolique (R) obtenu par hydrolyse à l'aide de lipase à partir des glycérides obtenus à partir de graines de Coriaria.
Les résultats obtenus avec ce substrat et avec Pichia ohmeri et Pichia stipitis respectivement sont présentés dans le Tableau I.

**Tableau I :**

| Incorporation de l'acide coriolique (R) Rapport pondéral de gamma-nonanolide : delta - décanolide | | |
|---|---|---|
| | 24 h | 48 h |
| P. ohmeri | 0,18 | 0,36 |
| P. stipitis | 2,89 | 2,63 |

(b) l'acide coriolique (S) obtenu par lipoxygénation d'acide linoléique (90 % Fluka) à l'aide de lipoxygénase de soja (Fluka) à pH 9, en utilisant de l'air ou de l'oxygène. Après terminaison de la réaction, une solution de cystéinate de sodium est ajoutée. On laisse le mélange réactionnel réagir à température ambiante jusqu'à ce que le test au peroxyde soit négatif. Le pH est ajusté à 4,5 avec de l'acide citrique et l'acide coriolique (S) ainsi obtenu est extrait par des solvants.
Les résultats obtenus sont présentés dans le Tableau II.

**Tableau II :**

| Incorporation de l'acide coriolique (S) Rapport pondéral de gamma-nonanolide : delta - décanolide | | |
|---|---|---|
| | 24 h | 48 h |
| P. ohmeri | 0,15 | 0,13 |
| P. stipitis | 0,19 | 0,53 |

(c) le mélange des hydroxy-acides obtenus comme suit : 50 g de graines de lin sont broyées en une farine fine, en prenant soin de s'assurer que la masse ne subit pas de chauffage excessif au cours de l'opération. La farine résultante est mise en suspension dans un litre de tampon borate pH 9 et agitée pendant une heure. 3 ml/l d'acide linoléique sont ajoutés et on fait passer de l'air ou de l'oxygène pendant 6 heures à une température de 10-15°C. Du cystéinate de sodium est ajouté et les hydroxy-acides sont extraits comme décrit ci-dessus.
Les résultats obtenus, exprimés en rapport pondéral de gamma-nonanolide aux lactones totales, sont présentés au Tableau III.

**Tableau III**

| Incorporation de lipoxygénates de farine de lin Rapport de gamma-nonanolide : lactones totales | | |
|---|---|---|
| | 24 h | 48 h |
| P. ohmeri | 0,06 | 0,18 |
| P. stipitis | 0,05 | 0,36 |

(d) le mélange des hydroxy-acides obtenus comme suit : on soumet 10 ml d'acide linoléique dans 100 ml de chlorure de méthylène, en présence de 1 g de chlorophylle, à l'oxydation à l'air tout en exposant au rayonnement solaire. Après terminaison de la réaction, la phase organique est extraite par du tampon borate pH 9. La phase aqueuse contenant les hydroperoxydes est alors traitée par du cystéinate de sodium et les hydroxy-acides sont récupérés par extraction au solvant de la solution acidifiée avec de l'acide citrique.

Les résultats obtenus, exprimés en rapports pondéraux de gamma-nonanolide aux lactones totales sont présentés dans le Tableau IV.

**Tableau IV :**

| Incorporation des produits de photo-oxydation Rapport de gamma-nonanolide : lactones totales | | |
|---|---|---|
| | 24 h | 48 h |
| P. ohmeri | 0,17 | 0,14 |
| P. stipitis | 0,66 | 0,16 |

## Revendications

1. Méthode de préparation de gamma-lactones ayant 9 atomes de carbone, saturées ou insaturées, ou leurs mélanges, sous forme naturelle **caractérisée en ce qu'**elle implique la culture de Pichia stipitis dans un substrat comprenant un hydroxy-acide insaturé en C₁₈, l'hydroxyle étant en C₁₃, ou un glycéride le contenant, pour obtenir un mélange comprenant le gamma-nonanolide.

2. Méthode selon la revendication 1, dans laquelle ledit substrat contient de l'acide coriolique (R).

3. Méthode selon la revendication 1, dans laquelle ledit substrat est le produit obtenu par photo-oxydation/réduction de l'acide linoléique ou un mélange d'acides gras saturés en C₁₈ comprenant l'acide linoléique.

4. Méthode selon la revendication 1, dans laquelle ledit substrat est le produit obtenu par lipoxygénation/réduction de l'acide linoléique ou un mélange d'acides gras insaturés en C₁₈ comprenant l'acide linoléique.

5. Méthode selon la revendication 4, dans laquelle la lipoxygénation est effectuée à l'aide d'une lipoxygènase naturelle présente dans la farine de lin.

6. Méthode selon la revendication 3 ou 4, dans laquelle la réduction du produit de photo-oxydation ou de lipoxygénation est effectuée à l'aide d'un agent de réduction naturel qui est le sulfate ferreux ou la cystéine ou l'un de ses sels.

## Claims

1. Method for preparing saturated or unsaturated gamma-lactones having 9 carbon atoms, or mixtures thereof, in a natural form, **characterized in that** it involves culturing Pichia stipitis in a substrate comprising an unsaturated C₁₈ hydroxy acid, the hydroxyl being a C₁₃, or a glyceride containing it, in order to obtain a mixture comprising gamma-nonanolide.

2. Method according to Claim 1, in which the said substrate contains (R)-coriolic acid.

3. Method according to Claim 1, in which the said substrate is the product obtained by photooxidation/reduction of linoleic acid or a mixture of saturated C₁₈ fatty acids comprising linoleic acid.

4. Method according to Claim 1, in which the said substrate is the product obtained by lipoxygenation/reduction of linoleic acid or a mixture of unsaturated C₁₈ fatty acids comprising linoleic acid.

5. Method according to Claim 4, in which the lipoxygenation is carried out with the aid of a natural lipoxygenase present in linseed meal.

6. Method according to Claim 3 or 4, in which the reduction of the product of photooxidation or of lipoxygenation is carried out with the aid of a natural reducing agent which is ferrous sulphate or cysteine or one of its salts.

## Patentansprüche

1. Verfahren zur Herstellung von gesättigten oder ungesättigten gamma-Lactonen mit 9 Kohlenstoffatomen oder deren Mischungen in natürlicher Form, **dadurch gekennzeichnet, dass** es die Kultur von Pichia stipitis in einem Substrat beinhaltet, welches eine ungesättigte C₁₈-Hydroxylsäure, wobei das Hydroxyl an C₁₃ vorliegt, oder ein Glycerid umfasst, das sie enthält, um eine Mischung zu erhalten, die gamma-Nonanolid umfasst.

2. Verfahren nach Anspruch 1, in dem das Substrat Coriolinsäure (R) enthält.

3. Verfahren nach Anspruch 1, in dem das Substrat das Produkt ist, das durch Photooxidation/Reduktion von Linolsäure oder einer Mischung von ungesättigten C₁₈-Fettsäuren, die Linolsäure umfasst, erhalten wird.

4. Verfahren nach Anspruch 1, in dem das Substrat das Produkt ist, das durch Lipoxygenierung/Reduktion von Linolsäure oder einer Mischung von ungesättigten C₁₈-Fettsäuren, die Linolsäure umfasst, erhalten wird.

5. Verfahren nach Anspruch 4, in dem die Lipoxygenierung mit Hilfe einer natürlichen, in Leinschrot vorhandenen Lipoxygenase bewirkt wird.

6. Verfahren nach Anspruch 3 oder 4, in dem die Reduktion des Produkts der Photooxidation oder der Lipoxygenierung mit Hilfe eines natürlichen Reduktionsmittels bewirkt wird, bei dem es sich um Eisen(II)-sulfat oder Cystein oder eines seiner Salze handelt.
